(19) Europäisches Patentamt
European Patent Office
Office européen des brevets

(11) Veröffentlichungsnummer: **0 057 363**
**B1**

(12) **EUROPÄISCHE PATENTSCHRIFT**

(45) Veröffentlichungstag der Patentschrift: **12.09.84**

(51) Int. Cl.³: **C 07 C 120/06,** C 07 C 121/45 // C07D213/80, C07D213/85

(21) Anmeldenummer: **82100303.5**

(22) Anmeldetag: **18.01.82**

(54) **Verfahren zur Herstellung von Butadiendinitrilen.**

(30) Priorität: **30.01.81 DE 3103066**

(43) Veröffentlichungstag der Anmeldung: **11.08.82 Patentblatt 82/32**

(45) Bekanntmachung des Hinweises auf die Patenterteilung: **12.09.84 Patentblatt 84/37**

(84) Benannte Vertragsstaaten: **BE CH DE FR GB IT LI NL**

(56) Entgegenhaltungen:
**DE - A - 2 424 373**

**ANGEWANDTE CHEMIE, Band 77, Nr. 5, 1965, Weinheim, H. BREDERECK et al. "Synthese und Reaktionen vinyloger Amidacetale und Amidine"**
**Chemical Abstracts, Band 69, Nr. 19, 4. November 1968, Columbus, Ohio, USA, R.A. FUICA et al. "3-(Disubstituted amino)acroleins. I. Condensations with malononitrile and ethyl cyanoacetate", Seite 8052, Spalte 1, Abstract Nr. 86279g**
**CHEMISCHE BERICHTE, Band 103, 1970, Weinheim, H. BREDERECK et al. "Reaktionen vinyloger Amidacetale, Aminalester und Amidaminale", Seiten 222 bis 235**

(73) Patentinhaber: **BASF Aktiengesellschaft, Carl-Bosch-Strasse 38, D-6700 Ludwigshafen (DE)**

(72) Erfinder: **Acker, Rolf-Dieter, Dr., Tuchbleiche 8, D-6906 Leimen (DE)**
Erfinder: **Hamprecht, Gerhard, Dr., Rote-Turm-Strasse 28, D-6940 Weinheim (DE)**

**EP 0 057 363 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft ein Verfahren zur Herstellung von Butadiendinitrilen durch Umsetzung von quaternären Ammoniumverbindungen mit Malodinitril in Gegenwart von Alkanolen und anschliessender Umsetzung mit Alkaliverbindungen.

Es ist bekannt, quaternäre Ammoniumverbindungen, die gleichzeitig Derivate des Malonaldehyds sind, der Formel

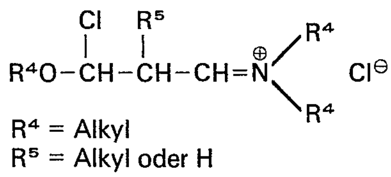

$R^4$ = Alkyl
$R^5$ = Alkyl oder H

durch die Umsetzung von Phosgen mit einem Dialkylformamid zum Dialkylformamidchlorid und anschliessender Umsetzung mit einem Enolether herzustellen (DE-OS Nr. 2424373).

Es ist weiterhin bekannt (Synthesis 1979, Seiten 376 bis 378), dass man Alkylidenmalodinitrile mit Lithium-diisopropylamid in Tetrahydrofuran bei −65° C und anschliessender Umsetzung mit Dimethylformamid-dichlorid zu 4-Dimethylamino-1,3-butadien-1,1-dicarbonitrile umsetzt. Diese Butadienverbindungen können durch Reaktion mit Ammoniak in Methanol zu 2-Aminopyridinen umgewandelt werden.

Es wurde nun gefunden, dass man Butadiendinitrile der Formel

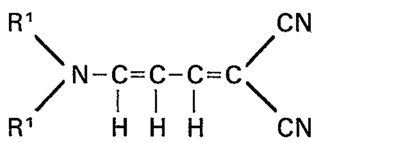 (I)

worin die Reste $R^1$ gleich oder verschieden sind und jeweils einen aliphatischen Rest bedeuten, vorteilhaft erhält, wenn man quaternäre Ammoniumverbindungen der Formel

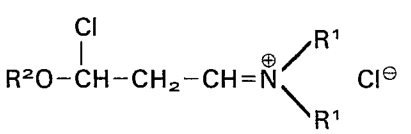 (II)

worin $R^1$ die vorgenannte Bedeutung besitzt und $R^2$ einen aliphatischen Rest bezeichnet,

a) mit Malodinitril in Gegenwart von Alkanolen umsetzt und dann

b) das Reaktionsgemisch mit Alkaliverbindungen umsetzt.

Die Umsetzung kann für den Fall der Verwendung von N,N-(3-Chlor-3-ethoxypropyliden)-N,N-dimethyl-ammoniumchlorid durch die folgenden Formeln wiedergegeben werden:

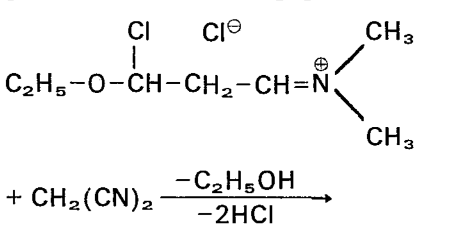


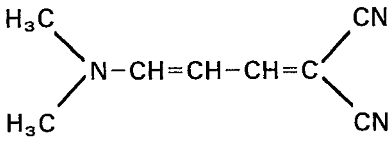

Im Hinblick auf die bekannten Verfahren liefert das Verfahren nach der Erfindung auf einfacherem und wirtschaftlicherem Wege Butadiennitrile in guter Ausbeute und Reinheit und besserer Gesamt-Raum-Zeit-Ausbeute. Schwer zugängliche Reaktionskomponenten wie Lithiumverbindungen werden vermieden. Alle diese vorteilhaften Ergebnisse sind im Hinblick auf den Stand der Technik überraschend.

Der Ausgangsstoff II ist leicht nach dem vorgenannten, in der DE-OS Nr. 2424373 beschriebenen Verfahren herstellbar. Er kann mit dem Malodinitril in stöchiometrischer Menge oder im Überschuss, zweckmässig in einem Verhältnis von 0,5 bis 3, vorzugsweise von 0,8 bis 1,5 Mol Malodinitril je Mol Ausgangsstoff II, umgesetzt werden. Bevorzugte Ausgangsstoffe II und dementsprechend bevorzugte Endstoffe I sind solche, in deren Formeln die Reste $R^1$ und $R^2$ gleich oder verschieden sind und jeweils einen Alkylrest mit 1 bis 7 Kohlenstoffatomen, vorzugsweise 1 bis 4 Kohlenstoffatomen, insbesondere Methyl und Ethyl, bedeuten. Die vorgenannten Reste können noch durch unter den Reaktionsbedingungen inerte Gruppen, z.B. Alkyl- oder Alkoxygruppen mit jeweils 1 bis 4 Kohlenstoffatomen, substituiert sein.

So sind beispielsweise als Ausgangsstoffe II geeignet: N,N-(3-Chlor-3-ethoxy-propyliden)-N,N-dimethyl-ammonium-chlorid; N,N-Diethyl-, N,N-Dipropyl-, N,N-Diisopropyl-, N,N-Dibutyl-, N,N-Diisobutyl-, N,N-Di-sek.-butyl-, N,N-Di-tert.-butylderivate; entsprechende 3-Methoxy-, 3-Propoxy-, 3-Isopropoxy-, 3-Butoxy-, 3-sek.-Butoxy-, 3-tert.-Butoxyderivate.

Die in Stufe a) verwendeten Alkanole enthalten zweckmässig 1 bis 10, insbesondere 1 bis 6 Kohlenstoffatome. Beispielsweise sind Methyl-, Ethyl-, Propyl-, Isopropyl-, Butyl-, Isobutyl-, sek.-Butyl-, tert.-Butyl-alkanol, bevorzugt Methanol, geeignet. Zweckmässig verwendet man von 1 bis 1000, insbesondere 10 bis 100 Mol Alkanol je Mol Ausgangsstoff II. Man setzt zweckmässig Malodinitril in einem Verhältnis 0,5 bis 3,0, vorzugsweise 0,5 bis 1,5 Mol je Mol Ausgangsstoff II um.

In der Stufe b) wird das Reaktionsgemisch mit Alkaliverbindungen, bevorzugt Alkalihydroxiden, Alkalicarbonaten und/oder Alkalialkoholaten, insbesondere Natrium-, Kaliumcarbonat, Natronlauge, Kalilauge, umgesetzt. Zweckmässig verwendet man gesättigte, wässerige Alkalicarbonatlösungen oder 0,1 bis 10 molare wässerige NaOH oder KOH und gibt gegebenenfalls noch Wasser zu. Insgesamt ist ein Verhältnis von 5 bis 500, insbesondere von 10 bis 100 Mol Wasser und/oder 0,2 bis 10, insbesondere von 0,5 bis 2 Äquivalente Alkaliverbindungen je Mol Ausgangsstoff II zweckmässig. Geeignete Alkaliverbindungen sind z.B. Kaliumhydroxid, Natriumhydroxid, Kaliumcarbonat, Natriumcarbonat, Lithiumhydroxid, Lithiumcarbonat, Natriumbicarbonat, Kaliumbicarbonat, Natriumformiat, Natriumacetat, Natriumpropionat, Natriumbutyrat, Natriumisobutyrat, Kaliumformiat, Kaliumacetat, Kaliumpropionat, Kaliumbutyrat, Kaliumbutyrat, Kaliumisobutyrat, Natriummethylat, Natriumethylat, Natriumpropylat, Natriumisopropylat, Natriumbutylat, Natriumisobutylat, Natrium-sek.-butylat, Natrium-tert.-butylat, Natriumäthylenglykolat, Natriumpropylen-(1,2)-glykolat, Natriumpropylen-(1,3)-glycolat, Natriumdiäthylenglykolat, Natriumtriäthylenglykolat, Natriumdipropylen-(1,2)-glykolat, Kaliummethylat, Kaliumethylat, Kalium-n-propylat, Kaliumisopropylat, Kalium-n-butylat, Kaliumisobutylat, Kalium-sek.-butylat, Kalium-tert.-butylat, Kaliumäthylenglykolat, Kaliumpropylen-(1,2)-glykolat, Kaliumdiäthylenglykolat, Kaliumtriäthylenglykolat, Kaliumdipropylen-(1,2)-glykolat.

In der Regel wird die Umsetzung in der Stufe a) bei einer Reaktionstemperatur je nach Alkanol von 40 bis 140, vorzugsweise 60 bis 120° C und in der Stufe b) bei einer Reaktionstemperatur von −10 bis +50, vorzugsweise −5 bis +30° C, drucklos oder unter Druck, kontinuierlich oder diskontinuierlich durchgeführt.

Die Reaktion kann wie folgt durchgeführt werden: Ein Gemisch von Ausgangsstoff II, Malodinitril und Alkanol wird während 2 bis 10 Stunden bei der Reaktionstemperatur der Stufe a) gehalten. Dann wird Sodalösung und Wasser zugesetzt und das Gemisch bei der Reaktionstemperatur der Stufe b) während 0,1 bis 3 Stunden gehalten. Der Endstoff kann in üblicher Weise, z.B. durch Extraktion, abgetrennt werden.

Die nach dem Verfahren der Erfindung erhältlichen Butadiennitrile sind wertvolle Ausgangsstoffe für die Herstellung von Farbstoffen, Schädlingsbekämpfungsmitteln, Pharmazeutika und Vitaminen. So können sie durch Umsetzung mit Ammoniak in Gegenwart von Alkanolen zu Aminonikotinnitrilen und aus diesen durch Umsetzung mit Alkali zu den Aminonikotinsäuren umgewandelt werden. Bezüglich der Verwendung wird auf die vorgenannten Veröffentlichungen und Ullmanns Enzyklopädie der technischen Chemie, Band 8, Seite 503; Band 14, Seite 480; Band 18, Seiten 195-198, verwiesen. Sie sind Ausgangsstoffe für die in der DE-OS Nr. 2430353 beschriebenen Schädlingsbekämpfungsmittel.

Die in den folgenden Beispielen aufgeführten Teile bedeuten Gewichtsteile.

*Beispiel 1*

a) 20 Teile N,N-(3-Chlor-3-ethoxy-propyliden)-N,N-dimethylammoniumchlorid, 6,6 Teile Malodinitril und 80 Teile Methanol werden 5 Stunden unter Rückfluss gekocht.

b) Nach dem Abdestillieren des Lösungsmittels weden 20 Teile Wasser zugegeben und mit gesättigter $Na_2CO_3$-Lösung auf pH=7 gestellt. Extraktion mit Methylenchlorid ergibt 13,1 Teile eines Öls, das nach Filtration 12,0 Teile (81% der Theorie) 1,1-Dicyano-4-N,N-dimethylamino-butadien-1,3 vom Fp 129 bis 132° C.

*Beispiel 2*

a) 220 Teile N,N-(3-Chlor-3-isobutoxy-propyliden)-N,N-dimethyl-ammoniumchlorid, 66 Teile Malodinitril und 800 Teile Methanol werden 6 Stunden unter Rückfluss bei ca. 66° C gekocht.

b) Nach dem Abdestillieren des Lösungsmittels werden 200 Teile Wasser zugegeben und mit gesättigter $Na_2CO_3$-Lösung auf pH=7 gestellt. Extraktion mit Methylenchlorid ergibt einen öligen Rückstand, der nach Zugabe von Methanol und unter Kühlung kristallisiert. Man erhält nach der Filtration 111 Teile (75% der Theorie) 1,1-Dicyano-4-N,N-dimethylamino-butadien-1,3.

*Beispiel 3*

Analog Beispiel 1 wird umgesetzt. Nach Stufe a) wird das Lösungsmittel abdestilliert, man fügt 30 Teile Wasser zu und stellt mit 2molarer Natronlauge auf pH=7. Extraktion mit Methylenchlorid und Kristallisation mit Methanol ergeben 11,5 Teile (77% der Theorie) 1,1-Dicyano-4-N,N-dimethylamino-butadien-1,3.

*Beispiel 4*

186 Teile N,N-(3-Chlor-3-methoxy-propyliden)-N,N-dimethylammoniumchlorid, 80 Teile Malodinitril und 500 Teile Methanol werden 4 Stunden unter Rückfluss gekocht.

Die Aufarbeitung entsprechend Beispiel 1 b) ergibt 123 Teile (84% der Theorie) 1,1-Dicyano-4-N,N-dimethylamino-butadien-1,3.

*Beispiel 5 (Verwendung)*

17,3 Teile 4-Dimethylamino-1,1-dicyano-butadien-1,3, 90 Teile Methanol werden in einem Rührautoklaven zusammengegeben. 16 Teile flüssiges Ammoniak werden zugefügt und die Mischung 3 Stunden bei 150° C gerührt. Die Mischung wird im Vakuum eingeengt, nach Zugabe von Ethanol wird der Niederschlag abgesaugt und getrocknet. Es ergeben sich 12,7 Teile 2-Aminonikotinnitril (91% der Theorie) vom Fp (aus Ethanol) 130 bis 132° C.

*Beispiel 6 (Verwendung)*

19,2 Teile 4-N,N-Diisobutylamino-1,1-dicyanobutadien-1,3, 80 Teile Methanol und 21 Teile Ammoniak werden entsprechend Beispiel 2 3 Stunden bei 150° C erhitzt. Nach dem Abkühlen wird das Lösungsmittel abdestilliert, 22 Teile Wasser und 3,7 Teile Natriumhydroxid werden zugeführt und 5 Stunden unter Rückfluss erhitzt. Nach dem Abkühlen wird der pH-Wert mit 10prozentiger HCl-Lösung auf 5 gebracht, abgekühlt und der Niederschlag abgetrennt. Es ergeben sich 8,1 Teile Amino-nikotinsäure (71% der Theorie) vom Fp 305 bis 310° C.

**Patentanspruch**

Verfahren zur Herstellung von Butadiendinitrilen der Formel

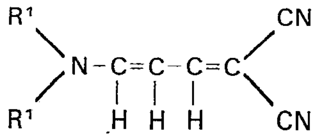 (I)

worin die Reste $R^1$ gleich oder verschieden sind und jeweils einen aliphatischen Rest bedeuten, dadurch gekennzeichnet, dass man quaternäre Ammoniumverbindungen der Formel

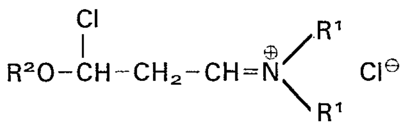 (II)

worin $R^1$ die vorgenannte Bedeutung besitzt und $R^2$ einen aliphatischen Rest bezeichnet,

a) mit Malodinitril in Gegenwart von Alkanolen umsetzt und dann

b) das Reaktionsgemisch mit Alkaliverbindungen umsetzt.

## Revendication

Procédé de préparation de dinitriles du butadiène de la formule

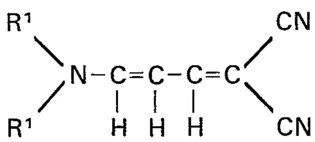 (I)

dans laquelle les restes $R^1$ peuvent être identiques ou différents et désigner chacun un groupe aliphatique, caractérisé en ce que

a) on fait réagir des dérivés d'ammonium quaternaire de la formule

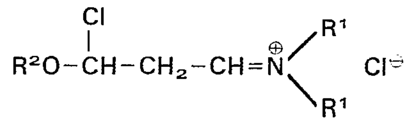 (II)

dans laquelle $R^1$ possède la signification définie et $R^2$ représente un groupe aliphatique, en présence d'alcanols avec le dinitrile malonique, puis

b) le mélange réactionnel avec des composés alcalins.

## Claim

A process for the preparation of a butadiene dinitrile of the formula

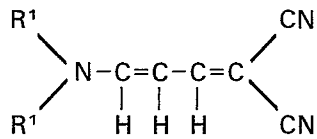 (I)

where the radicals $R^1$ are identical or different and each is an aliphatic radical, wherein a quaternary ammonium compound of the formula

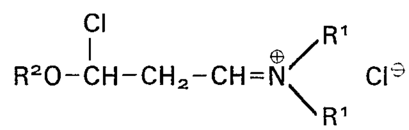 (II)

where $R^1$ has the above meaning and $R^2$ is an aliphatic radical,

a) is reacted with malodinitrile in the presence of an alkanol, and

b) the reaction mixture is then reacted with an alkali metal compound.